# EUROPÄISCHE PATENTSCHRIFT

(11) **EP 3 717 038 B1**
(45) Veröffentlichungstag und Bekanntmachung des Hinweises auf die Patenterteilung: **05.01.2022**
(21) Anmeldenummer: 18814804.3
(22) Anmeldetag: 26.11.2018
(51) Int. Cl.: A61M 1/16

(54) **DIALYSEGERÄT MIT VERWECHSLUNGSSICHERUNG FÜR HYDRAULIKANSCHLÜSSE**
DIALYSIS APPLIANCE WITH POKA-YOKE FOR HYDRAULIC PORTS
APPAREIL DE DIALYSE COMPORTANT UNE PROTECTION CONTRE L'INVERSION POUR LES RACCORDS HYDRAULIQUES

(30) Priorität: 28.11.2017 DE 102017128079
(43) Veröffentlichungstag der Anmeldung: 07.10.2020
(73) Patentinhaber: Fresenius Medical Care Deutschland GmbH, 61352 Bad Homburg (DE)
(72) Erfinder: HÄCKER, Jürgen, 61267 Neu-Anspach (DE); BOND, Oliver, 97520 Heidenfeld (DE); BREHM, Winfried, 97461 Hofheim (DE)
(74) Vertreter: Herrmann, Uwe
(86) Internationale Anmeldenummer: PCT/EP2018/082582
(87) Internationale Veröffentlichungsnummer: WO 2019/105895

(56) Entgegenhaltungen:
- EP-A1- 3 421 059
- DE-U1-202011 105 738
- US-A- 5 409 612
- US-A1- 2016 243 298

## Beschreibung

Die Erfindung betrifft ein Dialysegerät mit einem Zulaufanschluss zur Zuleitung frischer Dialysierflüssigkeit zum Dialysator, einem Ablaufanschluss zur Ableitung verbrauchten Dialysats vom Dialysator und zwei je einem Anschluss zugeordneten Parkstellen, wobei eine Verwechslungssicherung vorgesehen ist, die eine Lagerung des Zulaufanschluss und des Ablaufanschlusses an der jeweils zugehörigen Parkstelle sicherstellt.

Die Dialysatorschnittstelle ist eine fluidische Schnittstelle eines Dialysegeräts, an die erhöhte hygienische Anforderungen gestellt werden. Der Dialysator verfügt über zwei dialysatseitige Hydraulikanschlüsse. Einer dient als Zulauf für frische Dialysierflüssigkeit, der andere zur Abführung des verbrauchten Dialysats. Verbrauchtes Dialysat ist potentiell infektiös. Beim Abrüsten nach einer Behandlung gelangt Dialysat auf die Parkstellen bzw. -anschlüsse des Geräts. Über eine Farbkodierung wird derzeit angegeben, auf welche Parkstelle welcher Konnektor zu stecken ist (beispielsweise rot zu rot und blau zu blau). Tritt hier aufgrund fehlerhafter Handhabung eine Vertauschung auf, besteht die Gefahr, dass die Zulaufkupplung kontaminiert wird. Bei einer anschließenden Behandlung könnten dann pathogene Organismen auf die Hydraulikseite des Dialysators gelangen.

Die Druckschrift US2016/243298A1 offenbart eine Blutbehandlungsvorrichtung mit einem Dialysefilter oder einer Aufnahme für einen Dialysefilter, einer Dialysierflüssigkeitszulaufleitung mit einem ersten Verbinder zum Zuführen von Dialysierflüssigkeit zum Dialysefilter, einer Dialysatablaufleitung mit einem zweiten Verbinder zum Abführen von Dialysat vom Dialysefilter, einem Blutlecksensor konfiguriert und angeordnet zum Erkennen von Blut in oder am Dialysefilter, in der Dialysierflüssigkeitszulaufleitung und/oder in der Dialysatablaufleitung, und konfiguriert zum Abgeben eines Blutlecksignals (BLS) falls Blut erkannt wird, einer Kurzschlussleitung zum Verbinden der Dialysierflüssigkeitszulaufleitung und oder mit der Dialysatablaufleitung in Fluidkommunikation, wobei die Kurzschlussleitung hierzu einen dritten Verbinder und einen vierten Verbinder zum Verbinden mit jeweils einem der ersten und zweiten Verbinder aufweist, sowie einer Steuerung. Aus der US5409612A sind eine Vorrichtung und ein Verfahren zum Reinigen eines Dialysatkreislaufs stromabwärts eines Dialysators bekannt. Die Druckschrift DE202011105738 betrifft eine Online-Desinfektion eines Dialysegerätes, wobei es durch die Integrierung einer Diamant-Elektrolyse-Zelle (1) möglich wird In-Situ Oxidantien, resp. Desinfektionsmittel elektrolytisch zu generieren, die es ermöglichen, Dialyseräte (3) damit sehr wirkungsvoll zu desinfizieren.

Aufgabe der Erfindung ist es, ein Dialysegerät bereitzustellen, mit der eine solche Verwechslung sicher ausgeschlossen werden kann.

Die Erfindung wird durch die Merkmale des unabhängigen Anspruchs 1 definiert.

Vor diesem Hintergrund betrifft die Erfindung ein Dialysegerät mit zwei dialysatseitigen Hydraulikanschlüssen, an denen je eine Verbindungsleitung mit einer Filterkupplung zur Verbindung mit einem Dialysator angebunden ist, wobei es sich bei den Hydraulikanschlüssen um einen Zulaufanschluss zur Zuleitung frischer Dialysierflüssigkeit zum Dialysator und um einen Ablaufanschluss zur Ableitung verbrauchten Dialysats vom Dialysator handelt, sowie mit zwei den jeweiligen Hydraulikanschlüssen zugeordneten Parkstellen zum Lagern der Filterkupplungen bei Nichtbetrieb. Es ist vorgesehen, dass der Zulaufanschluss und die dem Ablaufanschluss zugeordnete Parkstelle im Abstand voneinander am Gerät angeordnet sind und die Länge der am Zulaufanschluss angebundenen Verbindungsleitung geringer als dieser Abstand ist, und dass der Ablaufanschluss und die dem Zulaufanschluss zugeordnete Parkstelle im Abstand voneinander am Gerät angeordnet sind und die Länge der am Ablaufanschluss angebundenen Verbindungsleitung geringer als dieser Abstand ist.

So ist es nicht möglich, die Filterkupplung des Zulaufanschlusses fälschlicherweise an die dem Ablaufanschluss zugeordnete Parkstelle zu setzen, und umgekehrt. Es werden also erfindungsgemäß die Positionen der Anschlüsse und Parkstellen sowie die Längen der Verbindungsleitungen in geeigneter Weise festgelegt, um eine Lagerung der Filterkupplungen an falschen Parkpositionen zu verhindern.

Erfindungsgemäß Z ist vorgesehen, dass der Zulaufanschluss an einer ersten Position und der Ablaufanschluss an einer zweiten Position am Gerät angeordnet sind, die in einem Abstand von der ersten Position liegt, der größer ist als die Länge sowohl der am Zulaufanschluss angebundenen Verbindungsleitung als auch der am Ablaufanschluss angebundenen Verbindungsleitung. Der Abstand ist aber nicht notwendigerweise größer als die Summe der Längen beider Verbindungsleitungen.

Erfindungsgemäß ist vorgesehen, dass der Zulaufanschluss und die dem Zulaufanschluss zugeordnete Parkstelle gemeinsam an einer ersten Position am Gerät angeordnet sind und dass der Ablaufanschluss und die dem Ablaufanschluss zugeordnete Parkstelle gemeinsam an einer zweiten Position am Gerät angeordnet sind, die in einem Abstand von der ersten Position liegt, der größer ist als die Länge sowohl der am Zulaufanschluss angebundenen Verbindungsleitung als auch der am Ablaufanschluss angebundenen Verbindungsleitung. Die Anschlüsse und zugeordneten Parkstellen sind also jeweils in unmittelbarer Nähe zueinander am Gerät angeordnet und bilden eine gemeinsame Station. Die Stationen sind im Abstand zueinander angeordnet, der so gewählt ist, dass weder die Filterkupplung des Zulaufanschlusses noch die Filterkupplung des Ablaufanschlusses an die falsche Parkstelle gesetzt werden können.

In einer Ausführungsform ist vorgesehen, dass das Dialysegerät eine Halterung für einen Dialysator aufweist, die so ausgebildet und am Gerät angeordnet ist, dass ein in die Halterung eingesetzter Dialysator räumlich zwischen dem Zulaufanschluss und dem Ablaufanschluss liegt. Dies bedeutet, dass der Abstand des Dialysators zu sowohl dem Zulaufanschluss als auch zu dem Ablaufanschluss geringer ist als der Abstand zwischen Zulaufanschluss und Ablaufanschluss. So können trotz der limitierten Länge der Verbindungsleitungen beide Filterkupplungen mit dem Dialysator verbunden werden.

Die Erfindung betrifft weiterhin eine Vorrichtung umfassend ein erfindungsgemäßes Dialysegerät und einen in einer dafür vorgesehenen Halterung des Dialysegeräts eingesetzten Dialysator, der einen Eingang für frische Dialysierflüssigkeit und einen im Abstand davon angeordneten Ausgang für verbrauchtes Dialysat aufweist, wobei der Abstand des Eingangs zum Ablaufanschluss größer als die Länge der am Ablaufanschluss angebundenen Verbindungsleitung ist und wobei der Abstand des Ausgangs zum Zulaufanschluss größer als die Länge der am Zulaufanschluss angebundenen Verbindungsleitung ist.

So kann darüber hinaus ein Anschluss der Filterkupplungen an der falschen Stelle am Dialysator verhindert werden. Selbstverständlich ist der Abstand des Eingangs zum Zulaufanschluss kleiner als die Länge der am Zulaufanschluss angebundenen Verbindungsleitung ist und der Abstand des Ausgangs zum Ablaufanschluss größer als die Länge der am Ablaufanschluss angebundenen Verbindungsleitung, sodass ein korrekter Anschluss stattfinden kann.

Weitere Einzelheiten und Vorteile der Erfindung ergeben sich aus dem nachfolgend anhand der Figuren beschriebenen Ausführungsbeispiel. In den Figuren zeigen:
- Figur 1:: eine Ansicht eines erfindungsgemäßen Dialysegeräts, wobei die Filterkupplungen der Hydraulikanschlüsse in den jeweiligen Parkstellen gelagert sind;
- Figur 2:: eine Ansicht desselben Dialysegeräts, wobei die Filterkupplungen der Hydraulikanschlüsse aus den jeweiligen Parkstellen entnommen sind;
- Figur 3:: eine Ansicht eines erfindungsgemäßen Dialysegeräts mit einem eingesetzten Dialysator, wobei die Filterkupplungen der Hydraulikanschlüsse nicht mit dem Dialysator gekoppelt sind; und
- Figur 4:: eine Ansicht desselben Dialysegeräts mit eingesetztem Dialysator, wobei die Filterkupplungen der Hydraulikanschlüsse mit dem Dialysator gekoppelt sind.

Die Figuren zeigen ein erfindungsgemäßes Dialysegerät 1, welches einen Zulaufanschluss 10 zur Zuleitung frischer Dialysierflüssigkeit zu einem Dialysator 30 und um einen Ablaufanschluss 20 zur Ableitung verbrauchten Dialysats vom Dialysator 30 umfasst.

Der Zulaufanschluss 10 ist mit einer ersten Verbindungsleitung 11 mit einer ersten Filterkupplung 12 versehen. Der Ablaufanschluss 20 ist mit einer zweiten Verbindungsleitung 21 mit einer ersten Filterkupplung 22 versehen. Das Gerät 1 umfasst des weiteren eine erste Parkstelle 13 für die erste Filterkupplung 12 des Zulaufanschlusses 10 sowie eine zweite Parkstelle 23 für die zweite Filterkupplung 22 des Ablaufanschlusses 20. Der Zulaufanschluss 10 und die erste Parkstelle 13 bilden eine gemeinsame Zulaufstation 14 am Gerät 1. Der Ablaufanschluss 20 und die zweite Parkstelle 23 bilden eine gemeinsame Ablaufstation 24 am Gerät 1.

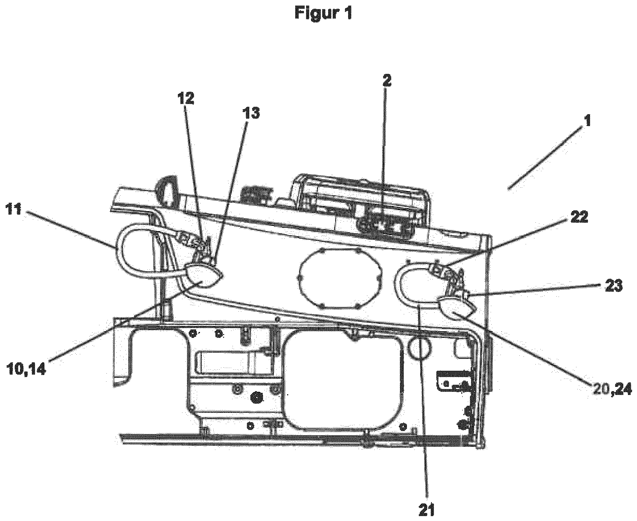

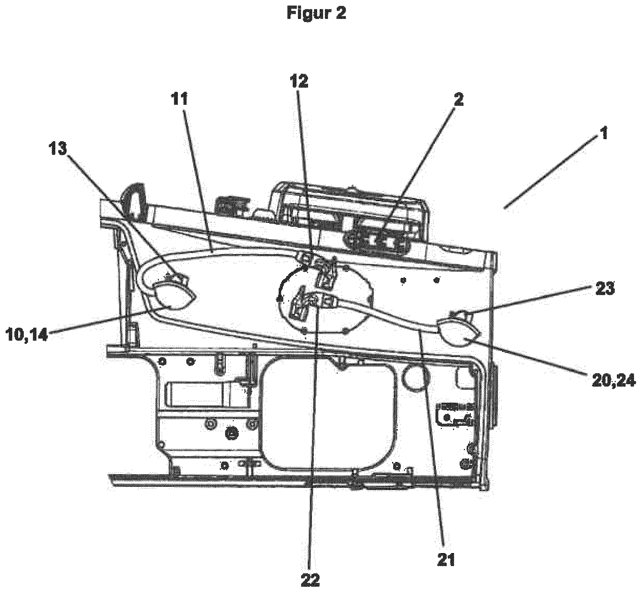

Die Zulaufstation 14 und die Ablaufstation 24 sind in einem Abstand zueinander angeordnet, der größer ist als die Länge sowohl der ersten Verbindungsleitung 11 als auch der zweiten Verbindungsleitung 21. Deshalb ist es unmöglich, die erste Filterkupplung 12 an die zweite Parkstelle 23 zu setzen, und auch unmöglich, die zweite Filterkupplung 22 an die erste Parkstelle 13 zu setzen, wie dies in der Figur 2 zu erkennen ist. Es ist lediglich möglich, die erste Filterkupplung 12 an die erste Parkstelle 13 zu setzen und die zweite Filterkupplung 22 an die zweite Parkstelle 23 zu setzen, wie dies in der Figur 1 zu erkennen ist.

Das Dialysegerät 1 umfasst ferner eine Halterung 2 zur Arretierung eines Dialysators 30 in einer definierten Position. Der Dialysator 30 umfasst einen an einem Ende des Dialysators 30 angeordneten Eingang 31 für frische Dialysierflüssigkeit und einen am gegenüberliegenden Ende des Dialysators 30 angeordneten Ausgang 32 für verbrauchtes Dialysat.

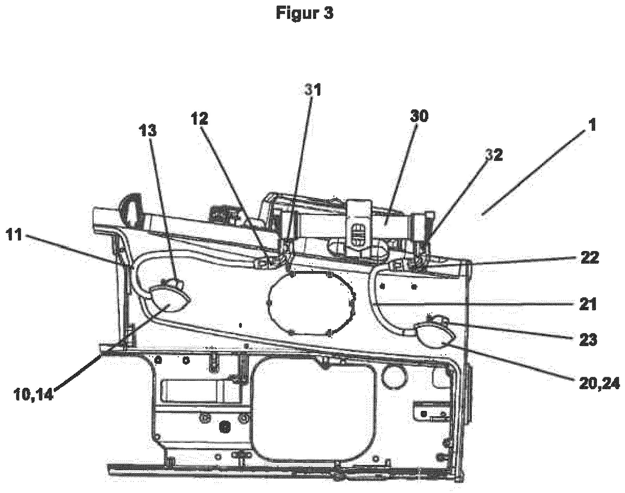

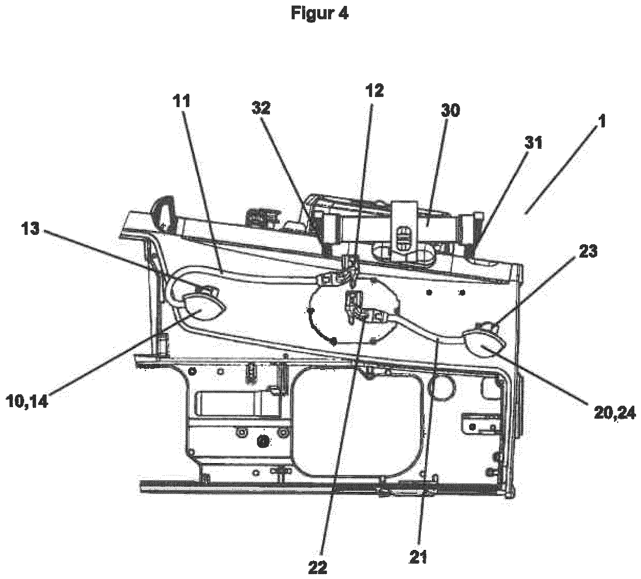

Die Halterung 2 liegt räumlich zwischen der Zulaufstation 14 und der Ablaufstation 24, was bedeutet, dass der Abstand des Dialysators zu sowohl der Zulaufstation 14 als auch der Ablaufstation 24 geringer ist als der Abstand zwischen der Zulaufstation 14 und der Ablaufstation 24. So können trotz der limitierten Länge der Verbindungsleitungen 11 und 21 beide Filterkupplungen 12 und 22 mit dem Dialysator verbunden werden, wie dies in Figur 3 dargestellt ist. Allerdings sind die Position des Dialysators 30 in der Halterung 2 und die Lage des Eingangs 31 und Ausgangs 32 am Dialysator so gewählt, dass der Abstand des Eingangs 31 zur Ablaufstation 24 größer als die Länge der am Ablaufanschluss 20 angebundenen Verbindungsleitung 21 ist und dass der Abstand des Ausgangs 32 zur Zulaufstation 14 größer als die Länge der am Zulaufanschluss 10 angebundenen Verbindungsleitung 11 ist, wie dies in Figur 4 dargestellt ist.

## Patentansprüche

1. Dialysegerät (1) mit zwei dialysatseitigen Hydraulikanschlüssen, an denen je eine Verbindungsleitung (11, 21) mit einer Filterkupplung (12, 22) zur Verbindung mit einem Dialysator (30) angebunden ist, wobei es sich bei den Hydraulikanschlüssen (10, 20) um einen Zulaufanschluss (10) zur Zuleitung frischer Dialysierflüssigkeit zum Dialysator und um einen Ablaufanschluss (20) zur Ableitung verbrauchten Dialysats vom Dialysator handelt, sowie mit zwei den jeweiligen Hydraulikanschlüssen zugeordneten Parkstellen (13, 23) zum Lagern der Filterkupplungen bei Nichtbetrieb,
**dadurch gekennzeichnet,**
**dass** der Zulaufanschluss (10) und die dem Ablaufanschluss (20) zugeordnete Parkstelle im Abstand voneinander am Gerät angeordnet sind und die Länge der am Zulaufanschluss angebundenen Verbindungsleitung geringer als dieser Abstand ist, und dass der Ablaufanschluss und die dem Zulaufanschluss zugeordnete Parkstelle im Abstand voneinander am Gerät angeordnet sind und die Länge der am Ablaufanschluss angebundenen Verbindungsleitung geringer als dieser Abstand ist, und dass der Zulaufanschluss und die dem Zulaufanschluss zugeordnete Parkstelle gemeinsam an einer ersten Position am Gerät angeordnet sind und dass der Ablaufanschluss und die dem Ablaufanschluss zugeordnete Parkstelle gemeinsam an einer zweiten Position am Gerät angeordnet sind, die in einem Abstand von der ersten Position liegt, der größer ist als die Länge sowohl der am Zulaufanschluss angebundenen Verbindungsleitung als auch der am Ablaufanschluss angebundenen Verbindungsleitung.

2. Dialysegerät nach einem der vorhergehenden Ansprüche, **dadurch gekennzeichnet, dass** das Dialysegerät eine Halterung für einen Dialysator aufweist, die so ausgebildet und am Gerät angeordnet ist, dass ein in die Halterung eingesetzter Dialysator räumlich zwischen dem Zulaufanschluss und dem Ablaufanschluss liegt.

3. Vorrichtung umfassend ein Dialysegerät nach Anspruch 2 und einen in der Halterung eingesetzten Dialysator, der einen Eingang für frische Dialysierflüssigkeit und einen im Abstand davon angeordneten Ausgang für verbrauchtes Dialysat aufweist, wobei der Abstand des Eingangs zum Ablaufanschluss größer als die Länge der am Ablaufanschluss angebundenen Verbindungsleitung ist und wobei der Abstand des Ausgangs zum Zulaufanschluss größer als die Länge der am Zulaufanschluss angebundenen Verbindungsleitung ist.

## Claims

1. A dialysis machine (1) having two hydraulic connectors at the dialyzate side to which a respective connection line (11, 21) having a filter coupling (12, 22) is connected for connection to a dialyzer (30), with the hydraulic connectors (10, 20) being an inflow connector (10) for feeding fresh dialysis liquid to the dialyzer and an outflow connector (20) for draining consumed dialyzate from the dialyzer, and having two parking positions (13, 23) associated with the respective hydraulic connectors for storing the filter couplings when not in operation,
**characterized in that**
the inflow connector (10) and the parking position associated with the outflow connector (20) are arranged spaced apart from one another at the machine and the length of the connection line connected to the inflow connector is smaller than this spacing, and that the outflow connector and the parking position associated with the inflow connector are arranged spaced apart from one another at the machine and the length of the connection line connected to the outflow connector is smaller than this spacing, and the inflow connector and the parking position associated with the inflow connector are arranged together at a first position at the machine, and the outflow connector and the parking position associated with the outflow connector are arranged together at a second position at the machine that is spaced apart from the first position, said spacing being larger than the length of both the connection line connected to the inflow connector and the connection line connected to the outflow connector.

2. A dialysis machine in accordance with one of the preceding claims, **characterized in that** the dialysis machine has a holder for a dialyzer that is configured and is arranged at the machine such that a dialyzer placed in the holder is spatially disposed between the inflow connector and the outflow connector.

3. An apparatus comprising a dialysis machine in accordance with claim 2 and a dialyzer that is placed in the holder and that has an input for fresh dialysis liquid and an output arranged at a spacing therefrom for consumed dialyzate, with the spacing of the input from the outflow connector being greater than the length of the connection line connected to the outflow connector and with the spacing of the output from the inflow connector being greater than the length of the connection line connected to the inflow connector.

## Revendications

1. Appareil de dialyse (1) comportant deux raccords hydrauliques côté dialysat, auxquels est respectivement reliée une conduite de liaison (11, 21) dotée d'un couplage à filtre (12, 22) pour la liaison avec un dialyseur (30), les raccords hydrauliques (10, 20) étant un raccord d'admission (10) pour l'alimentation de fluide de dialyse frais vers le dialyseur et un raccord d'écoulement (20) pour l'évacuation de dialysat usagé depuis le dialyseur, ainsi que deux points de rangement (13, 23) associés aux raccords hydrauliques respectifs pour loger les couplages à filtre dans l'état hors fonctionnement,
**caractérisé en ce que**
le raccord d'admission (10) et le point de rangement associé au raccord d'écoulement (20) sont disposés sur l'appareil à distance l'un de l'autre et la longueur de la conduite de liaison reliée au raccord d'admission est inférieure à cette distance, et **en ce que** le raccord d'écoulement et le point de rangement associé au raccord d'admission sont disposés sur l'appareil à distance l'un de l'autre et la longueur de la conduite de liaison reliée au raccord d'écoulement est inférieure à cette distance, et **en ce que** le raccord d'admission et le point de rangement associé au raccord d'admission sont disposés ensemble en une première position sur l'appareil et **en ce que** le raccord d'écoulement et le point de rangement associé au raccord d'écoulement sont disposés ensemble en une seconde position sur l'appareil, qui est à une distance de la première position, qui est supérieure à la longueur aussi bien de la conduite de liaison reliée au raccord d'admission que de la conduite de liaison reliée au raccord d'écoulement.

2. Appareil de dialyse selon l'une des revendications précédentes, **caractérisé en ce que** l'appareil de dialyse comporte un support pour un dialyseur, qui est conçu et disposé sur l'appareil de telle manière qu'un dialyseur placé dans le support se trouve du point de vue spatial entre le raccord d'admission et le raccord d'écoulement.

3. Dispositif comprenant un appareil de dialyse selon la revendication 2 et un dialyseur placé dans le support, qui comporte une entrée pour fluide de dialyse frais et une sortie pour dialysat usagé disposée à une distance de celle-ci, la distance de l'entrée au raccord d'admission étant supérieure à la longueur de la conduite de liaison reliée au raccord d'écoulement et la distance de la sortie au raccord d'admission étant supérieure à la longueur de la conduite de liaison reliée au raccord d'admission.
